# EUROPEAN PATENT SPECIFICATION

(11) **EP 1 562 505 B1**
(45) Date of publication and mention of the grant of the patent: **20.01.2010**
(21) Application number: 03781379.7
(22) Date of filing: 27.10.2003
(51) Int. Cl.: A61B 18/14

(54) **ELECTROSURGICAL GENERATOR AND METHOD WITH REMOVABLE FRONT PANEL HAVING REPLACEABLE ELECTRICAL CONNECTION SOCKETS AND ILLUMINATED RECEPTACLES**
ELEKTROCHIRURGISCHER GENERATOR MIT ENTFERNBARER FRONTPLATTE MIT ERSETZBAREN ELEKTRISCHEN VERBINDUNGSBUCHSEN UND BELEUCHTUNGSAUFNAHMEN SOWIE DAZUGEHÖRENDES VERFAHREN
GENERATEUR ELECTROCHIRURGICAL A PANNEAU AVANT AMOVIBLE EQUIPE DE DOUILLES DE CONNEXION ELECTRIQUE REMPLACABLES ET DE RECEPTACLES ECLAIRES ET PROCEDE ASSOCIE

(30) Priority: 18.11.2002 US 298707
(43) Date of publication of application: 17.08.2005
(60) Divisional application: 07112783.1
(73) Proprietor: CONMED CORPORATION, Utica, NY 13502-5994 (US)
(72) Inventor: GONNERING, Wayne, J., Littleton, CO 80128 (US)
(74) Representative: Menges, Rolf
(86) International application number: PCT/US2003/033791
(87) International publication number: WO 2004/045441

(56) References cited:
- EP-A- 0 895 318
- EP-A- 0 947 167
- DE-A- 10 026 256
- US-A- 4 131 328
- US-A- 5 141 444
- US-A- 5 573 424
- US-A- 6 068 627
- US-A- 6 132 262
- US-A1- 2002 009 920
- US-B1- 6 319 031
- US-B1- 6 325 639

## Description

The invention generally relates to electrosurgery, and more specifically, to an electrosurgical generator and a method according to the preambles of independent claims 1 and 19, respectively.

Electrosurgery involves applying relatively high voltage, radio frequency (RF) electrical power to tissue of a patient undergoing surgery, for the purpose of cutting the tissue, coagulating or stopping blood or fluid flow from the tissue, or cutting or coagulating the tissue simultaneously. The high voltage, RF electrical power is created by an electrosurgical generator, and the electrical power from the generator is applied to the tissue from an instrument or handpiece manipulated by a surgeon during the surgical procedure.

In monopolar electrosurgery, the handpiece includes a single active electrode. The active electrode is applied to the tissue, and the electrical energy travels from the generator, through a conductor to the handpiece, from the active electrode of the handpiece into the tissue of the patient, where the cutting, coagulating or simultaneous cutting and coagulating effect is achieved at the interface of the active electrode with the tissue. The electrical current is distributed into the patient, collected from the patient by a return electrode connected to the patient at a location remote from the surgical site, and is returned to the electrosurgical generator by an electrical conductor connected to the return electrode.

In bipolar electrosurgery, the handpiece generally takes the form of a forceps. The active electrode and the return electrode are attached at opposite ends of the arms of the forceps. Tissue is grasped between the active and return electrodes and the electrosurgical energy is transferred directly between the active and return electrodes through the tissue. Bipolar electrosurgery is generally used only for coagulating tissue, such as by squeezing a severed vessel and applying the electrosurgical energy to seal the end of the severed vessel.

The surgeon controls the power delivered to the handpiece by depressing a finger switch on the handpiece, or by stepping on a foot switch which is associated with the particular handpiece. Depressing a finger switch or stepping on a foot switch delivers an activation signal to the electrosurgical generator. The electrosurgical generator responds to the activation signal by delivering the high power electrosurgical energy to the handpiece with which the activation signal is associated.

Frequently during the surgical procedure, a surgeon will use different handpieces on an alternating, interchangeable basis. For example, the surgeon may use one monopolar handpiece for cutting, another monopolar handpiece with a different shaped active electrode for coagulating, and bipolar forceps for coagulating blood flow from severed vessels. In some complex surgical procedures, two or more surgeons may operate on the same patient at the same time at different surgical sites, using the same electrosurgical generator. To accommodate these situations, it is typical that the multiple handpieces are simultaneously connected to a single electrosurgical generator.

The electrosurgical generator typically includes a front control panel that has sockets into which prongs of electrical connector plugs of the handpieces are inserted, thereby connecting the handpieces to the electrosurgical generator. The front control panel also typically includes an electrical socket by which to connect a connection plug of the return electrode. In addition, the front control panel also usually provides a connector by which to connect a connection plug for the foot switch.

Over the normal course of using the electrosurgical generator, the connection plugs of the handpieces, return electrode and foot switch are inserted into and removed from the electrical connection sockets generator many times. At least one handpiece and one return electrode will be connected for each surgical operation, and it is not unusual that a surgeon may replace one or more of the handpieces or the return electrode during the course of a procedure, particularly a relatively lengthy procedure. Thus, over the lifetime of the electrosurgical generator, thousands of physical connections of the handpieces, return electrodes and foot switches will be made to the electrosurgical generator. Such usage results in considerable wear on the connection sockets of the electrosurgical generator. Typically, the handpieces and return electrodes are disposable, so the wear on their connection plugs is inconsequential. Because the usable lifetime of the functional components of the electrosurgical generator is greater than the typical usable lifetime of the electrical connection sockets in the front panel, it is typical that the front panel electrical connection sockets must be replaced periodically during the course of normal maintenance of and service on the electrosurgical generator.

Other electronic components of the electrosurgical generator are also subject to wear and periodic replacement. For example, it is typical to switch the high voltage RF electrosurgical power to the handpieces through output relays. The output relays are typically located behind the front panel within an enclosure or housing of the electrosurgical generator. Locating the output relays behind the front panel minimizes the possibility that the high voltage RF electrosurgical power will create unwanted electrical noise and other unwanted signal anomalies in the other relatively low voltage components of the electrosurgical generator, because the other relatively low voltage components of the electrosurgical generator are usually positioned within the housing at locations more remote from the front panel. The high voltage output relay operates each time the surgeon depresses an activation control button on one of the handpieces or steps on the foot switch. During the course of a single electrosurgical procedure, activations of the high voltage output relay may occur hundreds of times. The high voltage output relays may be operated tens or hundreds of thousands of times during the course of normal use of a typical electrosurgical generator. Such a large number of activations cause the high voltage output relay to become worn and potentially unreliable, thereby requiring the output relays to be replaced on a regular maintenance schedule.

In addition to periodically servicing the electrical connection sockets in the front panel and replacing the high voltage output relays located behind the front panel, there are many other reasons for providing convenient access to the electrical and electronic components of the electrosurgical generator located within the housing behind the front panel.

Adequate visibility of the front panel plug-receiving receptacles is difficult or impossible under the circumstances where the electrosurgical generator is used in a substantially darkened operating room. Minimally invasive surgery is performed in an almost completely darkened operating room. The surgical procedure progressing within the patient is pictured by a miniature camera inserted into the patient, and the surgeon and the surgical personnel observe the procedure on television-like display monitor located next to the patient in the operating room. Darkening the operating room enhances visualizing the procedure on the display monitor. In other circumstances, it is unusual for the operating room to be darkened so that intense illumination can be directed only on the surgical site. Illuminating the surgical site in this manner enhances the ability of the surgeon to observe the tissue and the progress of the surgical procedure. Under both of these circumstances involving darkening of the operating room, the control and operational features of the electrosurgical generator will be less visible. If it is necessary to replace the handpieces or the return electrode during the course of a surgical procedure in a darkened operating room, which is not unusual in relatively lengthy or complicated procedures, doing so is made difficult by the inability to clearly discern the receptacles for the connector plugs and the other operational and control features of the electrosurgical generator.

In the field of electrosurgical generators, invisible infrared light has been introduced into a plug-receiving receptacle on a front panel of the electrosurgical generator for the purpose of optically interrogating the type of plug and accessory or handpiece connected to the electrosurgical generator, as shown in US-A-6 068 627. Invisible infrared light will not illuminate the receptacle to facilitate connecting and disconnecting plugs in a darkened surgical operating room.

It is the object of the present invention to illuminate a receptacle of an electrosurgical generator according to the preamble of independent claim 1 and to provide a method according to the preamble of independent claim 19 so that it can be visualized in the darkened environment of an operating room to facilitate connecting and disconnecting a plug and a plug-receiving receptacle.

The object is achieved by the present invention by an electrosurgical generator and a method as defined in the characterizing clauses of independent claims 1 and 19, respectively. Embodiments of the present invention form the subject matter of the dependent claims.

The improvements of this invention involve illuminating plug-receiving receptacles located on the front panel of the housing of an electrosurgical generator to facilitate making quick and effective connections and disconnections of a plug in a darkened environment, while reducing the possibility of the closely-adjacent high voltage RF electrosurgical output power inducing unwanted noise and signal anomalies in other functional components of the electrosurgical generator or the camera and monitoring devices used in minimally invasive surgery. In addition, a combined feature allows for convenient and relatively rapid replacement and maintenance of worn electrical connection sockets on the front panel and replacement and maintenance of high voltage relays located behind the front panel in an electrosurgical generator, as well as relatively convenient and quick access to other internal components of the electrosurgical generator which are either connected to the front panel or located behind the front panel.

One aspect of the invention relates to illuminating the plug-receiving receptacle of the electrosurgical generator. A light emitter is connected to project visible light into the plug-receiving receptacle. An optical conductor or fiber is connected to the light emitter to conduct light to the light emitter. The non-electrical light emitter and optical fiber will not pick up electrical noise and anomalous signals generated by the high voltage RF electrosurgical power conducted through the plug-receiving receptacle. The functionality of the electrosurgical generator becomes less susceptible to noise and anomalous functionality created by such noise.

Another aspect of the invention in combination with the illumination feature involves improvements in accessing internal components within an electrosurgical generator. Access is achieved by removing the front panel from the housing, and as part of the act of removing the front panel from the housing, separating an electrical connection between an electrical connector member and the circuit board. The components attached to the front panel and within the electrosurgical generator adjacent to the front panel are easily accessed by removing the front panel. A plug-receiving receptacle permits the replacement of a socket-defining electrical connector member. An insert receptacle is attached to a housing panel, and a retainer is located at the rear end of the insert receptacle. The retainer contacts the connector member at the rear end of the insert receptacle to retain the connector member within the insert receptacle. Removing the retainer releases the connector member so that it can be replaced in the insert receptacle. A resilient electrical contact is connected to the circuit board within the housing at a position to contact an extension portion of the connector member. The resilient electrical contact provides bias force to establish an electrical and mechanical connection between the connector member and the other internal components of the electrosurgical generator. The bias force facilitates a good electrical connection and convenient separation.

A more complete appreciation of these and other aspects of the invention and its scope, and the manner in which the present invention achieves the above noted and other improvements, can be obtained by reference to the appended claims and the following detailed description of a presently preferred embodiment taken in connection with the accompanying drawings which are briefly summarized below.

### Brief Description of the Drawings

Fig. 1 is an external perspective view of an electrosurgical generator, and a typical finger-switched actuated monopolar electrosurgical handpiece, a typical foot-switched monopolar electrosurgical handpiece, a typical bipolar electrosurgical handpiece, a typical foot switch, and a typical return electrode, all of which may be connected to the electrosurgical generator.
Fig. 2 is an enlarged cross-sectional view of a front panel of an electrosurgical generator and some of the internal components of the electrosurgical generator shown in Fig. 1 taken substantially in the plane in of line 2-2 of Fig. 1.
Fig. 3 is an enlarged view of a portion of Fig. 2 illustrating details of an electrical connection socket and details of its electrical connection to a front portion of an electronic circuit board within the electrosurgical generator.
Fig. 4 is a partial rear perspective view of an assembled relationship of the front panel and a front portion the electronic circuit board of the electrosurgical generator shown in Figs. 1-3. with an outer cover of the enclosure of the electrosurgical generator removed for clarity.
Fig. 5 is an exploded rear perspective view of the front panel and the front portion of the electronic circuit board shown in Fig. 4.
Fig. 6 is an exploded rear perspective view of the front panel and components of the electrical connection socket retained by the front panel shown in Figs. 2 and 3.

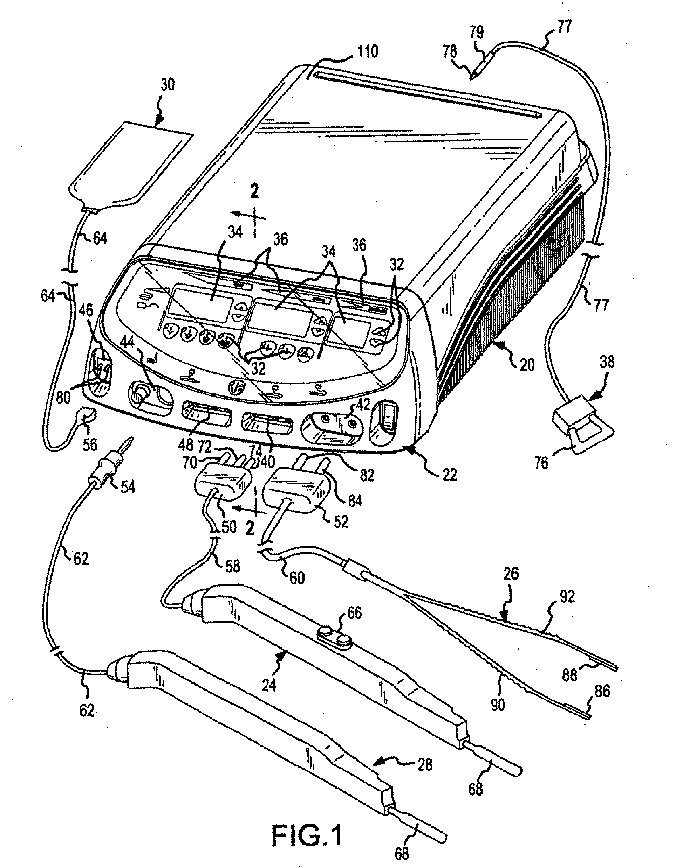

An electrosurgical generator 20, shown in Fig. 1, includes a front panel 22 at which to connect various conventional electrosurgical instruments and accessories, including a finger-switched monopolar handpiece 24, a bipolar handpiece or forceps 26, a foot-switched monopolar handpiece 28, and a return electrode 30. The front panel 22 also includes various touch input switch devices 32, displays 34 and indicators 36, which are used to control the operation of the electrosurgical generator by setting cut, coagulation or simultaneous cut and coagulation modes of electrosurgical operation and the amount of electrosurgical power to be delivered in the selected mode of operation, among other typical things. The front panel 22 functions as a user interface for the electrosurgical generator 20 with regard to performing input/output tasks. A typical foot switch 38 is also connected to the electrosurgical generator 20, at a back or rear housing panel (not visible in Fig. 1).

The front panel 22 locates and positions various electrical connector plug- receiving receptacles 40, 42, 44 and 46 by which to electrically connect the finger-switched monopolar handpiece 24, the bipolar forceps 26, the foot-switched monopolar handpiece 28 and the return electrode 30, respectively. The front panel 22 also includes another electrical connector plug-receiving receptacle 48 by which to connect an additional finger-switched monopolar handpiece (not shown) similar to the one handpiece 24 shown. The finger-switched monopolar handpiece 24, the bipolar forceps 26, the foot-switched monopolar handpiece 28 and the return electrode 30 each include an electrical connector plug 50, 52, 54 and 56 which is inserted into the plug-receiving receptacles 40, 42, 44 and 46 when connecting the finger-switched monopolar handpiece 24, the bipolar forceps 26, the foot-switched monopolar handpiece 28 and the return electrode 30 to the electrosurgical generator 20, respectively. The connector plugs 50, 52, 54 and 56 are electrically connected by conductors 58, 60, 62 and 64 to the finger-switched monopolar handpiece 24, the bipolar forceps 26, the foot-switched monopolar handpiece 28 and the return electrode 30, respectively.

The typical finger-switched monopolar handpiece 24 includes a finger activation switch 66 for the surgeon to depress to activate the electrosurgical generator 20 to deliver electrosurgical power from the plug-receiving receptacle 40 (or 48), to the connector plug 50, through the conductors 58 to the handpiece 24, and from an active electrode 68 connected at the distal end of the handpiece 24. One prong 70 of the connector plug 50 conducts the high voltage RF electrosurgical power through one of the conductors 58 to the active electrode 68. Two other prongs 72 and 74 of the connector plug 50 conduct activation signals from the activation switch 66 through the conductors 58 to the electrosurgical generator 20. The prong 72 conducts an activation signal from the finger switch 66 indicating that coagulation mode power is to be delivered to the active electrode 68, and the prong 74 conducts an activation signal from the finger switch 66 indicating that cut mode power is to be delivered to the active electrode 68.

The foot-switched monopolar handpiece 28 is similar to the finger-switched monopolar handpiece 24, except that the foot-switched monopolar handpiece 28 does not include a finger switch 66 to activate the electrosurgical generator 20. Instead, the foot-switched monopolar handpiece 28 requires the use of the foot switch 38 to activate the electrosurgical generator 20. The foot switch 38 includes a pedal 76 which is depressed by the foot of the surgeon, and in response, the foot switch 38 delivers an activation signal through conductors 77 to a prong 78 of a foot switch connector plug 79. In response to the activation signal from the foot switch 38, the electrosurgical generator 20 is activated and delivers electrosurgical power through the plug-receiving receptacle 44 into which is the electrical connector plug 54 of the foot-switched monopolar handpiece 28 is connected.

The electrical circuit for monopolar current flow from the active electrode 68 through the patient is established by connecting the return electrode 30 to the skin of the patient at a location remote from the surgical site. The electrical current is collected from the patient's body by the return electrode 30, conducted through the conductors 64 and returned to the electrosurgical generator through connector plug 56 which is inserted into the plug-receiving receptacle 46. The plug receiving receptacle 46 for connecting the return electrode 30 includes a pair of male prongs 80 which extend into female sockets (not shown) of the connector plug 56.

Electrical energy for bipolar electrosurgery performed with the bipolar forceps 26 is delivered from the plug-receiving receptacle 42 and conducted through prongs 82 and 84 of the connector plug 52. The electrosurgical energy is conducted from the plug-receiving receptacle 42 and into the prongs 82 and 84 of the connector plug 52, through the conductors 60 and delivered to electrodes 86 and 88 connected at the distal ends of arms 90 and 92 of the forceps 26. One of the electrodes 86 or 88 transfers current into the tissue confined between the two electrodes 86 and 88 by squeezing the arms 90 and 92 and the other electrode 86 or 88 collects and returns the current from the tissue. In bipolar electrosurgery, the electrosurgical current flows directly between the electrodes 86 and 88, making the use of the return electrode 30 unnecessary. The electrosurgical generator is typically activated to deliver the bipolar electrosurgical energy to the forceps 26 by depressing the pedal 76 of the foot switch 38, in the same manner as has been as has been described for activating the foot-switched monopolar handpiece 28. Some types of bipolar forceps 26 include a switch which generates an activation signal upon squeezing the arms 90 and 92 together.

Each time the electrosurgical generator 20 is set up for use in monopolar electrosurgery, the return electrode connector plug 56 and at least one monopolar handpiece connector plug 50 and/or 54 must be connected into the plug-receiving receptacles 40 (or 48) and/or 44, respectively. In addition, if a foot-switched monopolar handpiece 28 is connected, the foot switch connector plug 79 must be connected into a receptacle located on the back panel (not shown) of the electrosurgical generator 20. Each time the electrosurgical generator is set up for use in bipolar electrosurgery, the bipolar handpiece connector plug 52 must be inserted into the plug-receiving receptacle 42, and the foot switch connector plug 79 of the foot switch 38 must be inserted into the foot switch receptacle (not shown) on the back panel of the electrosurgical generator unless the bipolar forceps 26 are the type which have a switch which activates upon squeezing the arms 90 and 92. After the surgical procedure is completed, or when it is necessary to replace one of the handpieces 24, 26 or 28 or the return electrode 30 during the procedure, the connector plugs must be removed from the plug-receiving receptacles to disconnect the handpieces and other instruments from the electrosurgical generator. Over a normal course of use of the electrosurgical generator 20, these connections will be made and broken many times, perhaps thousands or tens of thousands of times.

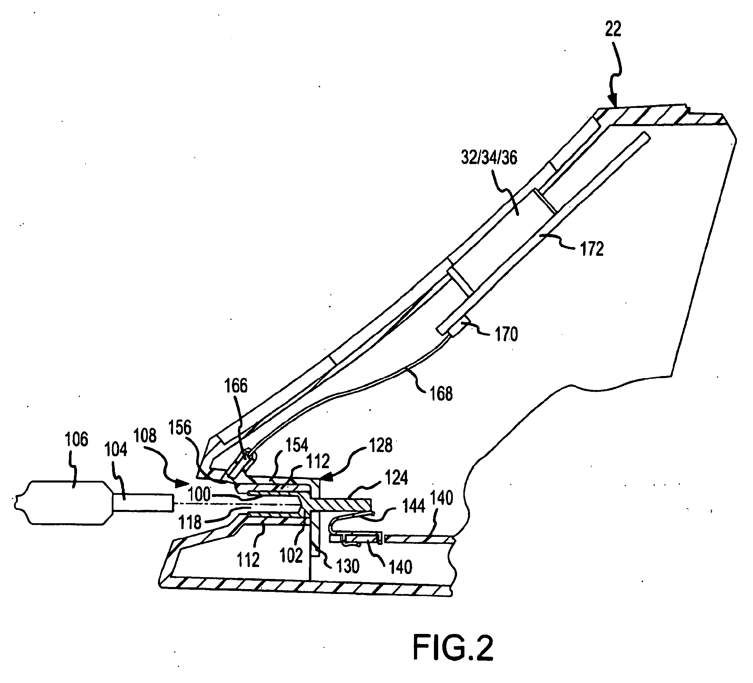

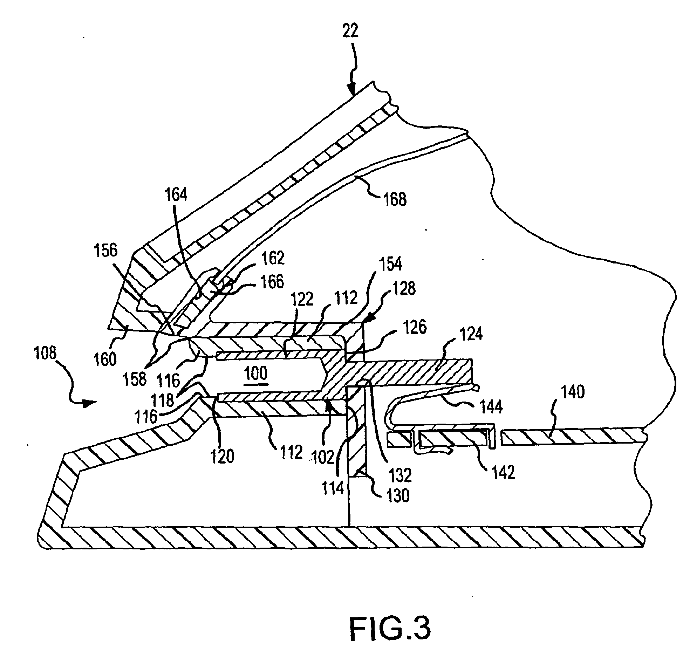

The repeated insertion and removal of the connector plugs into and from the plug-receiving receptacles creates significant wear on a socket 100 of a socket-defining connector member 102 which accepts and receives a prong 104 of a connector plug 106, as shown in Fig. 2. Each plug-receiving receptacle 40, 42 and 48 (Fig. 1) includes at least one connector member 102 which defines and provides the socket 100 for each of the prongs 70, 72, 74, 82 and 84 of the connector plugs 50 and 52 which are connected within the corresponding plug-receiving receptacle 40 (48) and 42 (Fig. 1), respectively. Although only a single prong 104 of a single connector plug 106 is illustrated in Fig. 2, the single prong 104 and connector plug 106 are representative of the prongs 70, 72, 74, 82 and 84 and connectors 50 and 52 illustrated in Fig. 1. In addition, Figs. 2 and 3 illustrate a single plug-receiving receptacle 108, which represents the plug receiving receptacles 40, 42 and 48 illustrated in Fig. 1. The foot switch plug-receiving receptacle 44 (Fig. 1) does not typically include a socket 100 or a socket-defininq member 102 (Fig. 2).

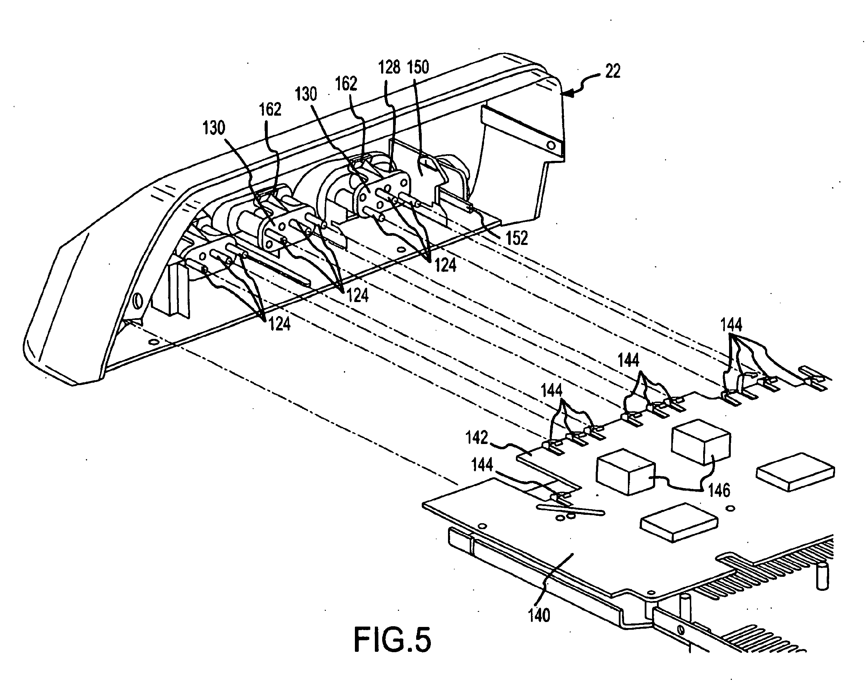

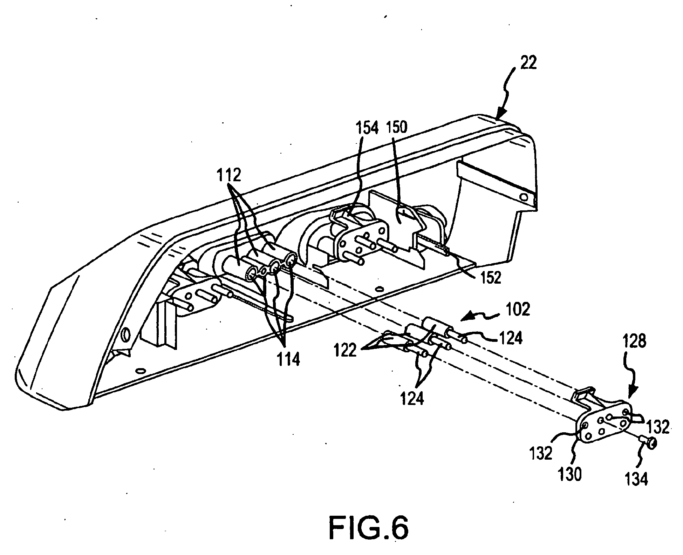

With reference to Fig. 2, after the socket 100 has been worn to such an extent that its dimensions are so large that an inadequate electrical connection may occur between the socket-defining member 102 and the prong 104, it is necessary to replace the socket-defining member 102 with one that has an appropriately sized socket 100 to establish a proper electrical connection with the prong 104 of the connector plug 106. To facilitate replacing worn socket-defining members 102 with enlarged sockets 100, the front panel 22 (Fig. 1) is made removable from an enclosure or housing 110 of the electrosurgical generator 20 (Fig. 1), as is generally shown in Figs. 5 and 6. In addition, connector members 102 which define the sockets 100 are made removable from the front panel 22 and the plug-receiving receptacles (40, 42 and 48, Fig. 1), as shown in Fig. 6, to facilitate their replacement. Moreover, the socket-defining connector members 102 are easily disconnected from the internal electrical components of the electrosurgical generator when the front panel 22 is removed from the housing 110, as shown in Fig. 5. As a result, the socket defining connection members 102 are easily replaceable and serviceable, as are other components attached to the front panel 22.

As shown in Figs. 2 and 3, the front panel 22 includes insert receptacles 112 which are molded or formed in the front panel 22 and which project rearwardly from the plug receptacle 108 into the interior of the electrosurgical generator. Each insert receptacle 112 is essentially a hollow tube-like structure, and one socket-defining connector member 102 is retained within each insert receptacle 112. Each socket-defining connector member 102 is inserted into the insert receptacle 112 from a rear open end 114 (Figs. 3 and 6) of the insert receptacle 112. As best shown in Fig. 3, a shoulder 116 is formed surrounding an opening 118 at the front end of each insert receptacle 112. The shoulder 116 contacts a forward edge 120 of the socket-defining connector member 102 to prevent the connector member 102 from moving forward out of the insert receptacle 112. The opening 118 provides access into the socket 100 of the connector member 102. As understood from Fig. 2, the prong 104 of the connector plug 106 is inserted through the opening 118 and into the socket 100.

Each socket-defining connector member 102 has a front cylindrical portion 122 within which the socket 100 is formed. The socket 100 is also preferably formed in a cylindrical configuration, and extends from the forward edge 120 rearwardly to a depth or distance which is approximately equal to or slightly greater in length as the typical length of the each prong 104 of the typical plug connector 106 (Fig. 2). A rear extenssion or shank portion 124 extends rearwardly from the rear of the front cylindrical portion 122. The shank portion 124 also has a cylindrical outer configuration, but is of smaller diameter than the larger front cylindrical portion 122. A shoulder 126 transitions between the larger diameter front cylindrical portion 122 and the smaller diameter rear shank portion 124. The shoulder 126 is located slightly rearwardly of the rearwardmost position of the socket 100. With the forward edge 120 of the socket-defining connector member 102 abutted against the shoulder 116, the shoulder 126 of the connector member 102 is approximately at the location of the rear open end 114 of the insert receptacle 112.

The entire socket-defining connector member 102 is preferably formed of good electrically conducting metallic material, such as brass, which is capable of providing a good electrical and frictional connection between the prong 104 and the socket 100. Because materials which offers the capability of a good frictional fit and electrical contact, such as brass, are somewhat soft, the socket 100 is susceptible to wear as a result of the repeated insertion and withdrawal of the prong 104. The susceptibility of the socket 100 to wear makes it necessary to periodically replace the socket-defining connecting member 102, usually during routine periodic maintenance of the electrosurgical generator.

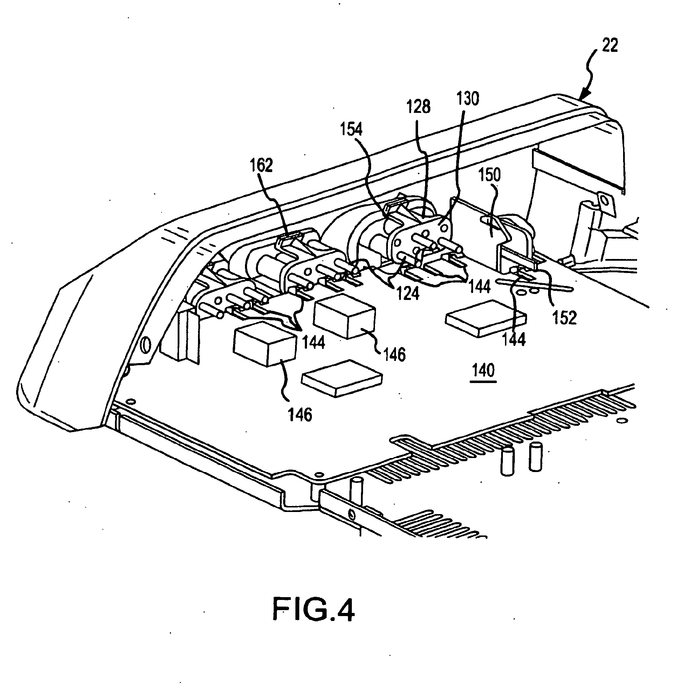

Each socket-defining connector member 102 is held in its insert receptacle 112 by a retainer 128. Each retainer 128 includes a back retaining panel 130 with a hole 132 through which the shank portion 124 of the connector member 102 extends. The retaining panel 130 is connected to the front panel 22 by screws or other fasteners 134 (Fig. 6). Connected in this manner, the retaining panel 130 of the retainer 128 contacts the shoulder 126 of the socket-defining connector member 102 to retain and trap the connector member 102 within the insert receptacle 112. The connector member 102 is held within the insert receptacle 112 because the front end 114 contacts the shoulder 116 at the front end of the connector member 102 and the shoulder 126 contacts the retaining panel 130 of the retainer 128. Although only a single hole 132 in the retaining panel 130 is illustrated in Figs. 2 and 3, each retaining panel 130 includes multiple holes 132 to accommodate the shank portions 124 of the multiple socket-defining connector members 102 in each of the plug receiving receptacles 40, 42 and 48 (Fig. 1) as is illustrated in Figs. 4-6.

The connection of each socket-defining connector member 102 to the front panel 22 in the manner described causes the rear shank portion 124 to extend rearwardly from the retaining panel 130. The rearwardly extending shank portion 124 electrically connects the socket-defining connector member 102 to an electrical circuit board 140 which is mounted within the enclosure 110 of the electrosurgical generator. The electrical circuit board 140 is retained within the housing 110 in such a way that its forward edge 142 is located adjacent to the front panel 22 when the front panel 22 is connected to the housing 110, as shown in Figs. 2-4. A resilient or leaf spring contact 144 is connected at the forward edge 142 of the circuit board 140 at a position which contacts the rearwardly extending shank portion 124 of each socket-defining connector member 102, when the front panel 22 is connected to the housing 110. One leaf spring contact 144 electrically connects each of the socket-defining connector members 102 to the circuit board 140.

The leaf spring contact 144 makes mechanical and electrical contact with the shank portion 124 of the connector member 102 as shown in Fig. 3. Upon contact, the shank portion 124 deflects the leaf spring contact 144 downwardly (as shown) to bias the leaf spring contact 144 against the shank portion 124. The spring bias force keeps the leaf spring contact 144 firmly engaged with the shank portion 124 to maintain a good electrical contact. An electrically conductive path is thus established from the socket-defining connector member 102 through the leaf spring contact 144 to the circuit board 140.

The leaf spring contacts 144 are electrically connected to conventional circuit conductors (not shown) formed on the circuit board 140. The circuit board electrical conductors conduct electrical signals between the various components of the circuit board 140 and the leaf spring contacts 144. For example, high voltage output relays 146 (Figs. 4 and 5) are connected to the circuit board 140 to deliver electrosurgical power through the leaf spring contacts 144 to the socket-defining connector members 102 for conduction to the handpieces 24 and 28 (Fig. 1). Signals from the activation switch 66 of the finger-switched monopolar handpiece 24 are also conducted through socket-defining connector members 102 to the circuit board 140, as a result of the electrical connection of the connector members 102 through the leaf spring contacts 144.

A foot-switched monopolar handpiece multi-plug connector device 150 is connected to the front panel 22 as shown in Figs. 4-6. The foot-switched monopolar multi-plug connector device150 includes a rearwardly extending electrical extension portion or contact member 152. The electrical contact member 152 projects rearwardly from the foot-switched monopolar multi-plug connector device 150 in a manner similar to the rearwardly projection of the shank portions 124 of the socket-defining connector members 102. A leaf spring contact 144 is connected near the front edge 142 of the circuit board 140 to make electrical contact with the contact member 152 of the foot-switched monopolar multi-plug connector device 150 when the front panel 22 is attached to the housing 110 of the electrosurgical generator. The contact member 152 depresses its mating leaf spring contact 144 in the same manner as the shank portions 124 depress their mating leaf spring contacts 144. Accordingly, the foot-switched monopolar multi-plug connector device 150 can be quickly and conveniently disconnected electrically from the internal components of the electrosurgical generator when the front panel 22 is removed.

The leaf spring contacts 144 permit the front panel 102 to be removed as a unit for convenience in servicing the electrosurgical generator 20 (Fig. 1), without having to disconnect the typical multiplicity of wires which extend between the circuit board 140 and the components attached to the front panel 22 in a conventional electrosurgical generator. The typical connection in a conventional electrosurgical generator involves soldering wires to the components of the front panel 22. Disconnecting the wires under such circumstances involves the relatively complicated and time-consuming process of melting those solder connections. The leaf spring contacts 144 greatly facilitate the convenience of gaining access to the front panel for replacing or servicing the components attached to the front panel 22, because the act of mechanically removing the front panel 22 from the housing 110 also electrically disconnects the front panel 22 from the circuit board 140 due to the separation of the shank portions 124 of the socket-defining connector members 102 and the electrical contact 152.of the foot-switched monopolar multi-plug connector device 150 from the leaf spring contacts 144. The main circuit board 140 remains connected to the housing 110 when the front panel 22 is removed.

With the front panel 22 removed from the housing 110 (Fig. 1) of the electrosurgical generator 20 (Fig. 1), it is relatively easy to replace a socket-defining connector member 102 by removing the screw 134 (Fig. 6) so that the retainer 128 can be disconnected from the front panel 22. Thereafter, the socket-defining connector member 102 is removed from the insert receptacle 112 and replaced with a new connector member 102. The retainer 128 is thereafter reconnected to hold the new connector member 102 in the insert receptacle 112. The front panel 22 is then reconnected to the housing 110, thereby re-establishing electrical contact between the shank portions 124 and the electrical contact 152 and their mating leaf spring contacts 144.

In addition to the convenience of replacing the socket-defining connector members 102 (Figs. 2 and 3), removing the front panel 22 provides convenient access to other internal components of the electrosurgical generator located at or near the front edge 142 of the circuit board 140 behind the front panel 22. It is typical to locate the high voltage output relays 146 in these positions. Replacing or servicing the high voltage relays 146 is more readily accomplished because of the space and access provided by the ability to remove the front panel 22. Moreover, replacing or servicing the foot-switched monopolar multi-plug connector device 150 is more readily accomplished because it is attached to the front panel 22 which is completely removable from the housing 110 of the electrosurgical generator. The foot-switched monopolar multi-plug connector device 150 can therefore be worked on or replaced without interference from other internal components of the electrosurgical generator.

Since the electrosurgical generator may be located in a darkened operating room with little or no illumination directed on the electrosurgical generator, the plug-receiving receptacles 40, 42, 44, 46 and 48 (Fig. 1) are illuminated to facilitate connecting the connector plugs 50, 52, 54 and 56 into those receptacles. Illuminating the receptacles and other components of the electrosurgical generator in this manner facilitates quick connections and disconnections of the handpieces, the return electrode and during the course of the surgery, if necessary, as well as control of the electrosurgical generator, in a darkened operating room.

A non-electrically powered light source is incorporated within each plug-receiving receptacle 40, 42, 44, 46 and 48, as illustrated the single receptacle 108 shown in Figs. 2 and 3 and as otherwise shown in Figs. 4-6. As shown primarily in Figs. 3 and 6, a bracket 154 is connected to an upper edge of the retaining panel 130 of the retainer 128. The bracket 154 projects forwardly from the retaining panel 130 to a position in front of the shoulder 116 at the insert receptacle 112, as shown in Fig. 3. A lens or transparent edge 156 is formed at the forward the end of the bracket 154. The transparent edge 156 is located above an opening 158 formed in an upper wall portion 160 of each receptacle 108 (Fig. 3). A retainer 162 is also formed at the forward end of the bracket 154 at a position above the transparent edge 156. The retainer 162 defines a slot 164 into which a light emitter 166 is positioned and held. Optical fibers 168 are connected to the light emitter 166, and the optical fibers 168 conduct light to the light emitter 166. The light emitter 166 delivers the light through the lens or transparent edge 156 to project into the receptacle 108. The light from the light emitter 166 illuminates the receptacle 108 making its features distinguishable in a darkened environment.

For those receptacles, e.g. 44 and 46 (Fig. 1), which do not include the removable socket-defining connector members 102, retainers 128 and brackets 154, a lens or transparent edge similar to that illustrated at 156 is located above an opening similar to that illustrated at 158 formed in an upper wall portion of each receptacle, e.g. 44 or 46 (Fig. 1). A retainer similar to that shown at 162 is also located above the transparent edge for receiving a light emitter similar to that illustrated at 166. Optical fibers to similar to those illustrated at 168 are connected to the light emitters to conduct light to the light emitters located above these other types of receptacles, e.g. 44 or 46 (Fig. 1).

The optical fibers 168 extend from the light emitter 166 to a source of light, for example, a light emitting diode 170, shown in Fig. 2. A light emitting diode 170 is mounted on a control panel circuit board 172, and the control panel circuit board 172 is connected to the backside of the front panel 22 (Fig. 2). Other components on the control panel circuit board 172 interact with and provide the input touch switch devices 32, displays 34 and indicators 36 (Fig. 1) which are visible and accessible on the front side of the front panel 22. The optical fibers 168 conduct the light created by the light emitting diode 172 to the light emitter 166. Conventional light pipes could be used as alternatives to the conventional optical fibers 168. Although not shown, the control panel circuit board 172 is connected by a multi-pin connector to the circuit board 140, so that disconnecting the single multi-pin connector makes it possible to completely electrically and mechanically disconnect the front panel 22 from the housing 110 of the electrosurgical generator. The return pad pins 80 (Fig. 1) within the receptacle 46 are also connected to the circuit board 140 with a conventional separable electrical connector.

The light emitters 166, fiber optic 168 and circuit board 172 are integral with the front panel 22 and are therefore removable with the front panel 22 when the panel is disconnected from the housing of the electrosurgical generator. Removing the front panel provides access to permit the socket-defining connector members 102 to be replaced or to permit the high voltage output relays 146 or other components on the circuit board 140 near the front edge 142 to be serviced or replaced.

If electrical conductors and conventional light sources were used in place of the light emitter 166 and the optical fibers 168, such electrical conductors could pick up electrical noise and anomalous signals created by the relatively high voltage RF electrosurgical power conducted through the closely adjacent socket-defining connector members 102. Such noise and anomalous signals could adversely affect the proper functionality of the other components of the electrosurgical generator connected to the control panel circuit board 172 and the main circuit board 140. By using optical fibers to supply the light to the receptacle 108, there are no electrical conductors associated with the light sources to pick up electrical noise and anomalous signals. The use of optical conductors as opposed to electrical conductors makes the electrosurgical generator more immune from the adverse influences of electrical noise and anomalous signals generated by the high voltage RF power delivered from the electrosurgical generator.

A presently preferred embodiment of the present invention and many of its advantages and improvements have been described above with a degree of particularity. Many other advantages and improvements will be apparent upon gaining a complete understanding of the present invention. The preferred embodiment of the invention has been described above, but the invention itself is defined by the scope of the following claims.

## Claims

1. An electrosurgical generator (20) which generates high-frequency, high-voltage electrosurgical electrical power, comprising:
a plug-receiving receptacle (108) at an exterior panel (22) of a housing (110) of the electrosurgical generator at which to transfer the electrosurgical power from the electrosurgical generator to an accessory (24, 26, 28, 30), the plug-receiving receptacle (108) including a socket (100) for receiving a prong (104) of a connector plug (106) connected to the accessory;
an electrically-energized source of light (170) located within the housing (110) at a position remote from the plug-receiving receptacle (108);
an optical conductor (168) extending from the light source (170) for conducting the light from the light source to the plug-receiving receptacle (108);
low-voltage electrical components (172) located within the housing (110);
**characterized by**:
the light source (170) producing visible light.

2. The electrosurgical generator (20) as defined in claim 1, further comprising:
a light emitter (166) positioned at the plug-receiving receptacle (108) to project the visible light from the optical conductor (168) onto the plug-receiving receptacle (108).

3. The electrosurgical generator (20) as defined in claim 1 or 2,
wherein:
the optical conductor comprises an optical fiber (168).

4. The electrosurgical generator (20) as defined in any one of the claims 1 to 3, wherein plug-receiving receptacle (108) comprises:
an insert receptacle (112) connected to the exterior panel (22) and having a front end connected to the plug-receiving receptacle (108) and extending rearwardly from the plug-receiving receptacle to a rear end (114), the front end of the insert receptacle (112) defining a front opening (118) to the plug-receiving receptacle;
a connector member (102) which defines the socket (100), the socket (100) extending from a front end (120) of the connector member toward a rear end (124) of the connector member, the connector member (102) removably positioned within the insert receptacle (112) with the front end (120) of the connector member positioned adjacent to and within the front opening (118) of the insert receptacle (112) with the socket (100) accessible through the front opening (118); and
a retainer (128) located at the rear end (114) of the insert receptacle (112) and contacting the connector member (102) at the rear end of the insert receptacle to retain the connector member within the insert receptacle.

5. The electrosurgical generator (20) as defined in claim 4, wherein:
the insert receptacle (112) includes a shoulder (116) surrounding the front opening (118) at the front end of the insert receptacle; and
the shoulder (116) of the insert receptacle (112) contacts the front end (120) of the connector member (102) to retain the connector within the insert receptacle.

6. The electrosurgical generator (20) as defined in claim 5, wherein:
the connector member (102) is insertable into and removable from the insert receptacle (112) at the rear end (114) of the insert receptacle, upon removal of the retainer (128).

7. The electrosurgical generator (20) as defined in claim 5, wherein:
the connector member (102) includes a shoulder (126) located adjacent to the rear end (114) of the insert receptacle (112); and
the retainer (128) contacts the shoulder (126) of the connector member.

8. The electrosurgical generator (20) as defined in claim 7, wherein:
the connector member (102) is confined within the insert receptacle (112) by contact of the front end (120) of the connector member with the shoulder (116) of the insert receptacle (112) and by contact of the shoulder (126) of the connector member with the retainer (128) at the rear end (114) of the insert receptacle (112).

9. The electrosurgical generator (20) as defined in claim 8, wherein:
the retainer (128) includes a retaining panel (130) having a hole (132) formed therethrough;
the connector member (102) includes a shank portion (124) extending beyond the rear end (114) of the insert receptacle (112); and
the retaining panel (130) is connected at the rear end (114) of the insert receptacle (112) with the hole (132) surrounding the shank portion (124).

10. The electrosurgical generator (20) as defined in claim 9, further comprising:
a circuit board (140) located behind the exterior panel (22); and
an electrical contact (144) connected to the circuit board (140) at a position to contact the shank portion (124) of the connector member (102) to establish an electrical connection between the circuit board (140) and the connector member (102).

11. The electrosurgical generator (20) as defined in claim 4, wherein:
the connector member (102) includes an extension portion (124,152) extending rearwardly beyond the rear end (114) of the insert receptacle (112); and further comprising:
a circuit board (140) located behind the exterior panel (22), and
an electrical contact (144) connected to the circuit board (140) at a position to contact the extension portion (124,152) of the connector member (102) to establish an electrical connection between the circuit board (140) and the connector member (102).

12. The electrosurgical generator (20) as defined in claim 11, wherein:
the electrical contact comprises a resilient member (144).

13. The electrosurgical generator (20) as defined in claim 12, wherein:
the resilient member comprises a leaf spring contact (144) which is deformed upon physical contact with the extension portion (124, 152).

14. The electrosurgical generator (20) as defined in claim 12, wherein:
the exterior panel (22) is connectable to and disconnectable from the housing (110); and
the extension portion (124, 152) separates from the resilient member (144) upon disconnection of the exterior panel (22) from the housing (110).

15. The electrosurgical generator (20) as defined in claim 14, wherein:
the resilient member(144) resiliently deflects to mechanically and electrically contact the extension portion (124, 152) upon connection of the exterior panel (22) to the housing (110) and mechanically separates from the extension member upon disconnection of the exterior panel from the housing.

16. The electrosurgical generator (20) as defined in claim 11, wherein:
the connector member (102) includes a forward portion (122) which defines the socket (100) and the front end (120), the forward portion (122) is located substantially within the insert receptacle (112), the extension portion (124) extends rearwardly from the forward portion (122), and the extension portion is of smaller cross-sectional size than the forward portion; and
the retainer (128) is connected at the rear end (114) of the insert receptacle (112) and contacts the extension portion (124) to confine the connector member (102) relative to the insert receptacle by contact of the front end (120) of the connector member with the shoulder (116) of the insert receptacle and by contact of the extension portion (124) with the retainer (128) at the rear end (114) of the insert receptacle.

17. The electrosurgical generator (20) as defined in claim 4, wherein:
the retainer (128) includes a bracket (154) which extends forwardly from the rear end (114) of the insert receptacle (112) to the front end (120) of the insert receptacle; and
the light emitter (166) is connected to a front end of the bracket (154) to project the light onto the plug-receiving receptacle (108).

18. The electrosurgical generator (20) as defined in claim 17, wherein:
the light emitter (166) is connected to the front end of the bracket (154) by a retaining portion (162) of the bracket; and
the light emitter (166) is removable from the retaining portion (162) of the bracket.

19. A method of projecting light onto a plug-receiving receptacle (108) at an exterior panel (22) of a housing (110) of an electrosurgical generator (20) which generates high-frequency, high-voltage electrosurgical electrical power, comprising:
transferring the electrosurgical power from a socket (100) of the plug-receiving receptacle (108) to a prong (104) of a connector plug (106) received within the socket in the plug-receiving receptacle, the plug (106) conducting the electrosurgical power to an accessory (24, 26, 28, 30) connected to the plug;
electrically energizing a source of light (170) within the electrosurgical generator (20) at a position remote from the plug-receiving receptacle (108);
projecting light from the light source (170) through a non- electrically conductive optical conductor (168) onto the plug-receiving receptacle (108);
**characterized by**:
the light source (170) emitting visible light.

20. The method as defined in claim 19, further comprising:
projecting the visible light from the optical conductor (168) onto the plug-receiving receptacle (108) with a light emitter (166).

21. The method as defined in claim 19 or 20, further comprising:
conducting the visible light from the light source (170) to the plug-receiving receptacle (108) through an optical fiber (168).

22. The method as defined in any one of the claims 19 to 21, further comprising:
using a connector member (102) to define the plug-receiving receptacle (108);
positioning the connector member (102) in an insert receptacle (112) which has a front end attached to the exterior panel (22) at a front opening (118) and a rear end (114) extending from the exterior panel (22) into the housing (110); and
retaining the connector member within the insert receptacle at both the front and rear ends of the insert receptacle.

23. The method as defined in claim 22, further comprising:
including a circuit board (140) within the electrosurgical generator behind the exterior panel (22);
extending an extension portion (124, 152) of the connector member (102) rearwardly beyond the rear end (114) of the insert receptacle (112): and
resiliently contacting the extension portion (124, 152) of the connector member (102) with the circuit board (140) to establish an electrical connection (144) between circuit board (140) and the connector member (102).

24. The method as defined in claim 23, further comprising:
removing the exterior panel (22) from the housing (110); and
separating the resilient contact (144) between the extension portion (124, 152) and the circuit board (140) as a part of the act of removing the exterior panel (22) from the housing (110).

25. The method as defined in claim 24, further comprising:
removing the connector member (102) from the insert receptacle (112) after removing the exterior panel (22) from the housing (110); and
inserting a different connector member (102) in the insert receptacle (112) while the exterior panel (22) is removed from the housing (110).

26. The method as defined in claim 24, further comprising:
applying spring bias force between the extension portion (124, 152) and the circuit board (140) to establish and maintain the electrical connection (144) between the circuit board (140) and the connector member (102).

27. The method as defined in claim 26, further comprising:
removing the spring bias force between the extension portion (124, 152) and the circuit board (140) when removing the exterior panel (22) from the housing (110).

## Patentansprüche

1. Elektrochirurgischer Generator (20), welcher elektrochirurgische elektrische Energie hoher Frequenz und hoher Spannung erzeugt, mit:
einer Steckdose (108) in einer äußeren Platte (22) eines Gehäuses (110) des elektrochirurgischen Generators, von welcher aus die elektrochirurgische Energie aus dem elektrochirurgischen Generator zu einem Zubehörteil (24, 26, 28, 30) übertragbar ist, wobei die Steckdose (108) eine Buchse (100) zum Empfangen eines Kontaktstiftes (104) eines mit dem Zubehörteil verbundenen Verbindungssteckers (106) aufweist;
einer elektrisch gespeisten Lichtquelle (170), die in dem Gehäuse (110) in einer von der Steckdose (108) entfernten Position angeordnet ist;
einem Lichtleiter (168), der sich von der Lichtquelle (170) aus erstreckt, um das Licht von der Lichtquelle zu der Steckdose (108) zu leiten; und
elektrischen Niederspannungsbauteilen (172), die in dem Gehäuse (110) angeordnet sind;
**dadurch gekennzeichnet, dass** die Lichtquelle (170) sichtbares Licht erzeugt.

2. Elektrochirurgischer Generator (20) nach Anspruch 1, weiter mit:
einem Lichtemitter (166), der an der Steckdose (108) angeordnet ist, um das sichtbare Licht aus dem Lichtleiter (168) auf die Steckdose (108) zu projizieren.

3. Elektrochirurgischer Generator (20) nach Anspruch 1 oder 2, wobei:
der Lichtleiter eine Lichtleitfaser (168) umfasst.

4. Elektrochirurgischer Generator (20) nach einem der Ansprüche 1 bis 3, wobei die Steckdose (108) aufweist:
eine Einsatzdose (112), die mit der äußeren Platte (22) verbunden ist und ein vorderes Ende hat, das mit der Steckdose (108) verbunden ist und sich von der Steckdose aus zu einem hinteren Ende (114) erstreckt, wobei das vordere Ende der Einsatzdose (112) eine vordere Öffnung (118) für die Steckdose bildet; ein Verbindungsteil (102), welches die Buchse (100) bildet, wobei sich die Buchse (100) von einem vorderen Ende (120) des Verbindungsteils zu einem hinteren Ende (124) des Verbindungsteils erstreckt, wobei das Verbindungsteil (102) in der Einsatzdose (112) lösbar positioniert ist, wobei das vordere Ende (120) des Verbindungsteils benachbart zu und innerhalb der vorderen Öffnung (118) der Einsatzdose (112) positioniert ist und wobei die Buchse (100) über die vordere Öffnung (118) zugänglich ist; und
einen Halter (128), der an dem hinteren Ende (114) der Einsatzdose (112) angeordnet ist und das Verbindungsteil (102) an dem hinteren Ende der Einsatzdose kontaktiert, um das Verbindungsteil in der Einsatzdose festzuhalten.

5. Elektrochirurgischer Generator (20) nach Anspruch 4, wobei:
die Einsatzdose (112) eine Schulter (116) aufweist, welche die vordere Öffnung (118) an dem vorderen Ende der Einsatzdose umgibt; und
die Schulter (116) der Einsatzdose (112) das vordere Ende (120) des Verbindungsteils (102) kontaktiert, um das Verbindungsteil in der Einsatzdose festzuhalten.

6. Elektrochirurgischer Generator (20) nach Anspruch 5, wobei:
das Verbindungsteil (102) in die Einsatzdose (112) an dem hinteren Ende (114) der Einsatzdose nach dem Entfernen des Halters (128) einsetzbar und aus derselben herausziehbar ist.

7. Elektrochirurgischer Generator (20) nach Anspruch 5, wobei:
das Verbindungsteil (102) eine Schulter (126) aufweist, die an dem hinteren Ende (114) der Einsatzdose (112) angeordnet ist; und
der Halter (128) die Schulter (126) des Verbindungsteils kontaktiert.

8. Elektrochirurgischer Generator (20) nach Anspruch 7, wobei:
das Verbindungsteil (102) in die Einsatzdose (112) durch Kontakt des vorderen Endes (120) der Verbindungsteils mit der Schulter (116) der Einsatzdose (112) und durch Kontakt der Schulter (126) des Verbindungsteils mit dem Halter (128) an dem hinteren Ende (114) der Einsatzdose (112) eingeschlossen ist.

9. Elektrochirurgischer Generator (20) nach Anspruch 8, wobei:
der Halter (128) eine Halteplatte (130) aufweist, in der ein Durchgangsloch (132) gebildet ist;
das Verbindungsteil (102) einen Schaftteil (124) aufweist, der sich über das hintere Ende (114) der Einsatzdose (112) hinaus erstreckt; und
die Halteplatte (130) an das hintere Ende (114) der Einsatzdose (112) angeschlossen ist, wobei das Loch (132) den Schaftteil (124) umgibt.

10. Elektrochirurgischer Generator (20) nach Anspruch 9, weiter mit:
einer Leiterplatte (140), die hinter der äußeren Platte (22) angeordnet ist; und
einem elektrischen Kontakt (144), der mit der Leiterplatte (140) in einer Position verbunden ist, in welcher er den Schaftteil (124) des Verbindungsteils (102) kontaktiert, um eine elektrische Verbindung zwischen der Leiterplatte (140) und dem Verbindungsteil (102) herzustellen.

11. Elektrochirurgischer Generator (20) nach Anspruch 4, wobei:
das Verbindungsteil (102) einen Fortsatzteil (124, 152) aufweist, der sich nach hinten über das hintere Ende (114) der Einsatzdose (112) hinaus erstreckt; und weiter mit:
einer Leiterplatte (140), die hinter der äußeren Platte (22) angeordnet ist, und einem elektrischen Kontakt (144), der mit der Leiterplatte (140) in einer Position verbunden ist, in welcher er den Fortsatzteil (124, 152) des Verbindungsteils (102) kontaktiert, um eine elektrische Verbindung zwischen der Leiterplatte (140) und dem Verbindungsteil (102) herzustellen.

12. Elektrochirurgischer Generator (20) nach Anspruch 11, wobei:
der elektrische Kontakt ein elastisches Teil (144) umfasst.

13. Elektrochirurgischer Generator (20) nach Anspruch 12, wobei:
das elastische Teil einen Blattfederkontakt (144) umfasst, welcher bei körperlichem Kontakt mit dem Fortsatzteil (124, 152) verformt wird.

14. Elektrochirurgischer Generator (20) nach Anspruch 12, wobei:
die äußere Platte (22) mit dem Gehäuse (110) verbindbar und von demselben trennbar ist; und
der Fortsatzteil (124, 152) sich von dem elastischen Teil (144) bei dem Trennen der äußeren Platte (22) von dem Gehäuse (110) trennt.

15. Elektrochirurgischer Generator (20) nach Anspruch 14, wobei:
das elastische Teil (144) sich elastisch biegt, um den Fortsatzteil (124, 152) bei dem Verbinden der äußeren Platte (22) mit dem Gehäuse (110) mechanisch und elektrisch zu kontaktieren, und sich bei dem Trennen der äußeren Platte von dem Gehäuse von dem Fortsatzteil mechanisch trennt.

16. Elektrochirurgischer Generator (20) nach Anspruch 11, wobei:
das Verbindungsteil (102) einen vorderen Teil (122) aufweist, der die Buchse (100) und das vordere Ende (120) bildet, wobei der vordere Teil (122) im Wesentlichen innerhalb der Einsatzdose (112) angeordnet ist, der Fortsatzteil (124) sich von dem vorderen Teil (122) aus nach hinten erstreckt und der Fortsatzteil eine kleinere Querschnittsgröße als der vordere Teil hat; und
der Halter (128) mit dem hinteren Ende (114) der Einsatzdose (112) verbunden ist und den Fortsatzteil (124) kontaktiert, um das Verbindungsteil (102) relativ zu der Einsatzdose durch Kontakt des vorderen Endes (120) des Verbindungsteils mit der Schulter (116) der Einsatzdose und durch Kontakt des Fortsatzteils (124) mit dem Halter (128) an dem hinteren Ende (114) der Einsatzdose einzuschließen.

17. Elektrochirurgischer Generator (20) nach Anspruch 4, wobei:
der Halter (128) eine Konsole (154) aufweist, die sich von dem hinteren Ende (114) der Einsatzdose (112) aus zu dem vorderen Ende (120) der Einsatzdose nach vorn erstreckt; und
der Lichtemitter (166) mit einem vorderen Ende der Konsole (154) verbunden ist, um Licht auf die Steckdose (108) zu projizieren.

18. Elektrochirurgischer Generator (20) nach Anspruch 17, wobei:
der Lichtemitter (166) mit dem vorderen Ende der Konsole (154) durch einen Halteteil (162) der Konsole verbunden ist; und
der Lichtemitter (166) aus dem Halteteil (162) der Konsole entfernbar ist.

19. Verfahren zum Projizieren von Licht auf eine Steckdose (108) in einer äußeren Platte (22) eines Gehäuses (110) eines elektrochirurgischen Generators (20), der elektrochirurgische elektrischen Energie hoher Frequenz und hoher Spannung erzeugt, durch:
Übertragen der elektrochirurgischen Energie von einer Buchse (100) der Steckdose (108) aus zu einem Kontaktstift (104) eines Verbindungssteckers (106), der in der Buchse in der Steckdose aufgenommen ist, wobei der Stecker (106) die elektrochirurgische Energie zu einem Zubehörteil (24, 26, 28, 30) leitet, das mit dem Stecker verbunden ist;
elektrisches Speisen einer Lichtquelle (170) innerhalb des elektrochirurgischen Generators (20) in einer von der Steckdose (108) entfernten Position; Projizieren von Licht aus der Lichtquelle (170) über einen nichtelektrisch leitfähigen Lichtleiter (168) auf die Steckdose (108);
**dadurch gekennzeichnet, dass** die Lichtquelle (170) sichtbares Licht emittiert.

20. Verfahren nach Anspruch 19, weiter beinhaltend:
Projizieren des sichtbaren Lichtes aus dem Lichtleiter (168) auf die Steckdose (108) mit einem Lichtemitter (166).

21. Verfahren nach Anspruch 19 oder 20, weiter beinhaltend:
Leiten des sichtbaren Lichtes aus der Lichtquelle (170) zu der Steckdose (108) über eine Lichtleitfaser (168).

22. Verfahren nach einem der Ansprüche 19 bis 21, weiter beinhaltend:
Verwenden eines Verbindungsteils (102) zum Bilden der Steckdose (108); Positionieren des Verbindungsteils (102) in einer Einsatzdose (112), die ein vorderes Ende hat, das an der äußeren Platte (22) in einer vorderen Öffnung (118) befestigt ist, und ein hinteres Ende (114), dass sich von der äußeren Platte (22) aus in das Gehäuse (110) erstreckt; und
Festhalten des Verbindungsteils in der Einsatzdose sowohl an dem vorderen als auch an dem hinteren Ende der Einsatzdose.

23. Verfahren nach Anspruch 22, weiter beinhaltend:
Vorsehen einer Leiterplatte (140) innerhalb des elektrochirurgischen Generators hinter der äußeren Platte (22);
Erstrecken eines Fortsatzteils (124, 152) des Verbindungsteils (102) nach hinten über das hintere Ende (114) der Einsatzdose (112) hinaus; und
elastisches Kontaktieren des Fortsatzteils (124, 152) des Verbindungsteils (102) mit der Leiterplatte (140) um eine elektrische Verbindung (144) zwischen der Leiterplatte (140) und dem Verbindungsteil (102) herzustellen.

24. Verfahren nach Anspruch 23, weiter beinhaltend:
Entfernen der äußeren Platte (22) von dem Gehäuse (110); und
Trennen des elastischen Kontakts (144) zwischen dem Fortsatzteil (124, 152) und der Leiterplatte (140) als einen Teil des Vorgangs des Entfernens der äußeren Platte (22) von dem Gehäuse (110).

25. Verfahren nach Anspruch 24, weiter beinhaltend:
Entfernen des Verbindungsteils (102) aus der Einsatzdose (112) nach dem Entfernen der äußeren Platte (22) von dem Gehäuse (110); und
Einsetzen eines anderen Verbindungsteils (102) in die Einsatzdose (112), während die äußere Platte (22) von dem Gehäuse (110) entfernt ist.

26. Verfahren nach Anspruch 24, weiter beinhaltend:
Ausüben einer Federvorspannkraft zwischen dem Fortsatzteil (124, 152) und der Leiterplatte (140), um die elektrische Verbindung (144) zwischen der Leiterplatte (140) und dem Verbindungsteil (102) herzustellen und aufrechtzuerhalten.

27. Verfahren nach Anspruch 26, weiter beinhaltend:
Entfernen der Federvorspannkraft zwischen dem Fortsatzteil (124, 152) und der Leiterplatte (140), wenn die äußere Platte (22) von dem Gehäuse (110) entfernt wird.

## Revendications

1. Générateur électro-chirurgical (20) qui génère une puissance électrique électro-chirurgicale à haute fréquence et haute tension, comprenant :
une prise de réception de fiche (108) au niveau d'un panneau extérieur (22) d'un boîtier (110) du générateur électro-chirurgical au niveau duquel transférer la puissance électro-chirurgicale provenant du générateur électro-chirurgical à un accessoire (24, 26, 28, 30), la prise de réception de fiche (108) comprenant une prise femelle (100) pour recevoir une fiche mâle (104) d'un connecteur (106) raccordé à l'accessoire ;
une source de lumière alimentée électriquement (170) située à l'intérieur du boîtier (110) à une position à distance de la prise de réception de fiche (108) ;
un conducteur optique (168) s'étendant à partir de la source de lumière (170) pour conduire la lumière de la source de lumière jusqu'à la prise de réception de fiche (108) ;
des composants électriques basse tension (172) situés à l'intérieur du boîtier (110) ; **caractérisé par** :
la source de lumière (170) produisant la lumière visible.

2. Générateur électro-chirurgical (20) selon la revendication 1, comprenant en outre :
un émetteur de lumière (166) positionné au niveau de la prise de réception de fiche (108) pour projeter la lumière visible du conducteur optique (168) sur la prise de réception de prise (108).

3. Générateur électro-chirurgical (20) selon la revendication 1 ou 2, dans lequel :
le conducteur optique comprend une fibre optique (168).

4. Générateur électro-chirurgical (20) selon l'une quelconque des revendications 1 à 3, dans lequel la prise de réception de fiche (108) comprend :
un réceptacle d'insert (112) raccordé au panneau extérieur (22) et ayant une extrémité avant raccordée à la prise de réception de fiche (108) et s'étendant vers l'arrière de la prise de réception de fiche jusqu'à une extrémité arrière (114), l'extrémité avant du réceptacle d'insert (112) définissant une ouverture avant (118) sur la prise de réception de fiche ;
un élément de connecteur (102) qui définit la fiche femelle (100), la fiche femelle (100) s'étendant à partir d'une extrémité avant (120) de l'élément de connecteur vers une extrémité arrière (124) de l'élément de connecteur, l'élément de connecteur (102) étant positionné de manière amovible à l'intérieur du réceptacle d'insert (112) avec l'extrémité avant (120) de l'élément de connecteur positionnée de manière adjacente à et à l'intérieur de l'ouverture avant (118) du réceptacle d'insert (112) avec la fiche femelle (100) qui est accessible par l'ouverture avant (118) ; et
un dispositif de retenue (128) situé au niveau de l'extrémité arrière (114) du réceptacle d'insert (112) et en contact avec l'élément de connecteur (102) au niveau de l'extrémité arrière du réceptacle d'insert pour retenir l'élément de connecteur à l'intérieur du réceptacle d'insert.

5. Générateur électro-chirurgical (20) selon la revendication 4, dans lequel :
le réceptacle d'insert (112) comprend un épaulement (116) entourant l'ouverture avant (118) au niveau de l'extrémité avant du réceptacle d'insert ; et
l'épaulement (116) du réceptacle d'insert (112) est en contact avec l'extrémité avant (120) de l'élément de connecteur (102) pour retenir le connecteur à l'intérieur du réceptacle d'insert.

6. Générateur électro-chirurgical (20) selon la revendication 5, dans lequel :
l'élément de connecteur (102) peut être inséré dans et retiré du réceptacle d'insert (112) au niveau de l'extrémité arrière (114) du réceptacle d'insert, suite au retrait du dispositif de retenue (128).

7. Générateur électro- chirurgical (20) selon la revendication 5, dans lequel :
l'élément de connecteur (102) comprend un épaulement (126) positionné de manière adjacente à l'extrémité arrière (114) du réceptacle d'insert (112) ; et
le dispositif de retenue (128) est en contact avec l'épaulement (126) de l'élément de connecteur.

8. Générateur électro-chirurgical (20) selon la revendication 7, dans lequel :
l'élément de connecteur (102) est confiné à l'intérieur du réceptacle d'insert (112) par le contact de l'extrémité avant (120) de l'élément de connecteur avec l'épaulement (116) du réceptacle d'insert (112) et par le contact de l'épaulement (126) de l'élément de connecteur avec le dispositif de retenue (128) au niveau de l'extrémité arrière (114) du réceptacle d'insert (112).

9. Générateur électro-chirurgical (20) selon la revendication 8, dans lequel :
le dispositif de retenue (128) comprend un panneau de retenue (130) ayant un trou (132) formé à travers celui-ci. ;
l'élément de connecteur (102) comprend une partie de tige (124) s'étendant au-delà de l'extrémité arrière (114) du réceptacle d'insert (112) ; et
le panneau de retenue (130) est raccordé au niveau de l'extrémité arrière (114) du réceptacle d'insert (112) avec le trou (132) qui entoure la partie de tige (124).

10. Générateur électro-chirurgical (20) selon la revendication 9, comprenant en outre :
une carte de circuit imprimé (140) positionnée derrière le panneau extérieur (22) ; et
un contact électrique (144) raccordé à la carte de circuit imprimé (140) au niveau d'une position pour entrer en contact avec la partie de tige (124) de l'élément de connecteur (102) afin d'établir une connexion électrique entre la carte de circuit imprimé (140) et l'élément de connecteur (102).

11. Générateur électro-chirurgical (20) selon la revendication 4, dans le lequel :
l'élément de connecteur (102) comprend une partie d'extension (124, 152) s'étendant vers l'arrière au-delà de l'extrémité arrière (114) du réceptacle d'insert (112) ; et comprenant en outre :
une carte de circuit imprimé (140) située derrière le panneau extérieur (22), et
un contact électrique (144) raccordé à la carte de circuit imprimé (140) à une position pour entrer en contact avec la partie d'extension (124, 152) de l'élément de connecteur (102) afin d'établir une connexion électrique entre la carte de circuit imprimé (140) et l'élément de connecteur (102).

12. Générateur électro-chirurgical (20) selon la revendication 11, dans lequel :
le contact électrique comprend un élément élastique (144).

13. Générateur électro-chirurgical (20) selon la revendication 12, dans lequel :
l'élément élastique comprend un contact à ressort à lames (144) qui est déformé suite au contact physique avec la partie d'extension (124, 152).

14. Générateur électro-chirurgical (20) selon la revendication 12, dans lequel :
le panneau extérieur (22) peut être raccordé à et se déconnecté du boîtier (110) ; et
la partie d'extension (124, 152) se sépare de l'élément élastique (144) suite à la déconnexion du panneau extérieur (22) du boîtier (110).

15. Générateur électro-chirurgical (20) selon la revendication 14, dans lequel :
l'élément élastique (144) dévie de manière élastique pour entrer en contact mécaniquement et électriquement avec la partie d'extension (124, 152), suite au raccordement du panneau extérieur (22) sur le boîtier (110) et se sépare mécaniquement de l'élément d'extension suite à la déconnexion du panneau extérieur du boîtier.

16. Générateur électro-chirurgical (20) selon la revendication 11, dans lequel:
l'élément de connecteur (102) comprend une partie avant (122) qui définit la fiche femelle (100) et l'extrémité avant (120), la partie avant (122) est positionnée sensiblement à l'intérieur du réceptacle d'insert (112), la partie d'extension (124) s'étend vers l'arrière à partir de la partie avant (122), et la partie d'extension a une taille transversale plus petite que la partie avant ; et
le dispositif de retenue (128) est raccordé à l'extrémité arrière (114) du réceptacle d'insert (112) et en contact avec la partie d'extension (124) pour confiner l'élément de connecteur (102) par rapport au réceptacle d'insert par le contact de l'extrémité avant (120) de l'élément de connecteur avec l'épaulement (116) du réceptacle d'insert et par le contact de la partie d'extension (124) avec le dispositif de retenue (128) au niveau de l'extrémité arrière (114) du réceptacle d'insert.

17. Générateur électro-chirurgical (20) selon la revendication 4, dans lequel :
le dispositif de retenue (128) comprend un support (154) qui s'étend vers l'avant à partir de l'extrémité arrière (114) du réceptacle d'insert (112) jusqu'à l'extrémité avant (120) du réceptacle d'insert ; et
l'émetteur de lumière (166) est raccordé à une extrémité avant du support (154) pour projeter la lumière sur la prise de réception de fiche (108).

18. Générateur électro-chirurgical (20) selon la revendication 17, dans lequel:
l'émetteur de lumière (166) est raccordé à l'extrémité avant du support (154) par une partie de retenue (162) du support; et
l'émetteur de lumière (166) peut être retiré de la partie de retenue (162) du support.

19. Procédé pour projeter de la lumière sur une prise de réception de fiche (108) au niveau d'un panneau extérieur (22) d'un boîtier (110) d'un générateur électro-chirurgical (20) qui génère une puissance électrique électro-chirurgical à haute fréquence et haute tension, comprenant les étapes consistant :
transférer la puissance électro-chirurgical d'une fiche femelle (100) de la prise de réception de fiche (108) à une fiche male (104) d'un connecteur (106) reçue à l'intérieur de la fiche femelle dans la prise de réception de fiche, la fiche (106) conduisant la puissance électro-chirurgical jusqu'à un accessoire (24, 26, 28, 30) raccordé à la fiche ;
alimenter électriquement une source de lumière (170) dans le générateur électro-chirurgical (20), à une position à distance de la prise de réception de fiche (108);
projeter la lumière de la source de lumière (170) par l'intermédiaire d'un conducteur optique non électriquement conducteur (168) sur la prise de réception de fiche (108) ;
**caractérisé par** :
la source de lumière (170) émettant la lumière visible.

20. Procédé selon la revendication 19, comprenant en outre l'étape consistant à :
projeter la lumière visible du conducteur optique (168) sur la prise de réception de fiche (108) avec un émetteur de lumière (166).

21. Procédé selon la revendication 19 ou 20, comprenant en outre l'étape consistant à :
conduire la lumière visible de la source de lumière (170) de la prise de réception de fiche (108) par une fibre optique (168).

22. Procédé selon l'une quelconque des revendications 19 à 21, comprenant en outre les étapes consistant à :
utiliser un élément de connecteur (102) pour définir la prise de réception de fiche (108) ;
positionner l'élément de connecteur (102) dans un réceptacle d'insert (112) qui a une extrémité avant fixée sur le panneau extérieur (22) au niveau d'une ouverture avant (118) et une extrémité arrière (114) s'étendant à partir du panneau extérieur (22) dans le boîtier (110) ; et
retenir l'élément de connecteur à l'intérieur du réceptacle d'insert à la fois au niveau des extrémités avant et arrière du réceptacle d'insert.

23. Procédé selon la revendication 22, comprend en outre les étapes consistant à :
inclure une carte de circuit imprimé (140) dans le générateur électro-chirurgical derrière le panneau extérieur (22) ;
étendre une partie d'extension (124, 152) de l'élément de connecteur (102) vers l'arrière au-delà de l'extrémité arrière (114) du réceptacle d'insert (112) ; et
mettre en contact de manière élastique la partie d'extension (124, 152) de l'élément de connecteur (102) avec la carte de circuit imprimé (140) pour établir une connexion électrique (144) entre la carte de circuit imprimé (140) et l'élément de connecteur (102).

24. Procédé selon la revendication 23, comprenant en outre les étapes consistant à :
retirer le panneau extérieur (22) du boîtier (110) ; et
séparer le contact élastique (144) entre la partie d'extension (124, 152) et la carte de circuit imprimé (140) comme faisant partie de l'acte consistant à retirer le panneau extérieur (22) du boîtier (110).

25. Procédé selon la revendication 24, comprenant en outre les étapes consistant à :
retirer l'élément de connecteur (102) du réceptacle d'insert (112) après avoir retiré le panneau extérieur (22) du boîtier (110) ; et
insérer un élément de connecteur (102) différent dans le réceptacle d'insert (112) alors que le panneau extérieur (22) est retiré du boîtier (110).

26. Procédé selon la revendication 24, comprenant en outre l'étape consistant à :
appliquer une force de sollicitation de rappel entre la partie d'extension (124, 152) et la carte de circuit imprimé (140) pour établir et maintenir la connexion électrique (144) entre la carte de circuit imprimé (140) et l'élément de connecteur (102).

27. Procédé selon la revendication 26, comprenant en outre l'étape consistant à :
retirer la force de sollicitation de rappel entre la partie d'extension (124, 152) et la carte de circuit imprimé (140) lorsque l'on retire le panneau extérieur (22) du boîtier (110).
